# EUROPEAN PATENT APPLICATION

(11) **EP 0 952 450 A1**
(43) Date of publication of application: **27.10.1999**
(21) Application number: 98402797.9
(22) Date of filing: 12.11.1998
(51) Int. Cl.: G01N 33/569, G01N 33/543, G01N 33/58, C07K 14/175

(54) **Diagnostic reagent for Puumala virus infection**

(30) Priority: 24.04.1998 KR 1479698
(71) Applicant: Lee, Ho Wang, Chongno-ku, Seoul 110-510 (KR); KOREA GREEN CROSS CORPORATION, Yongin-City, Kyungki-Do 449-900 (KR)
(72) Inventor: Lee, Ho Wang, Chongno-ku, Seoul, 110-510 (KR); Ryu, Sun Ja, Songpa-ku, Seoul 138-200 (KR); Ahn, Chang Nam, Suwon City, Kyungki-do 442-372 (KR); Kim, Hoon, Suwon-city, Kyungki-do, 442-190 (KR); Tomiyama, Tetsuo, Nerima-ku, Tokyo 178 (JP)
(74) Representative: Des Termes, Monique

(57) **Abstract**

A simple and effective diagnostic reagent for detecting Puumala virus infection is disclosed. The reagent for detecting Puumala virus infection by contacting a serum from a subject suspected to have Puumala virus infection and observing agglutination to determine whether the serum contains antibodies against Puumala virus is provided, wherein said reagent comprises sensitized water-insoluble carrier particles which have Puumala virus antigens bound to the outer surface thereof in an amount of 0.005 to 2 wt%.

## Description

### BACKGROUND OF THE INVENTION

### 1. FIELD OF THE INVENTION

The present invention relates to a diagnostic reagent for detecting virus infection. More particularly, it relates to a diagnostic reagent for serologically detecting Puumala virus infection with enhanced specificity and sensitivity, allowing a precise diagnosis of hemorrhagic fever with renal syndrome caused by Puumala virus infection.

### 2. DESCRIPTION OF THE PRIOR ARTS

Hantaviruses are serologically-related members of the family *Bunyaviridae*. The prototype hantavirus is Hantaan virus which infects the Asian striped field mouse *Apodemus agratius* and was discovered in 1976 (Lee *et al*., 'Isolation of the Etiologic Agent of Korean Hemorrhagic Fever,' J. Infect. Dis. 137:298-307(1978)). When the virus is transmitted from mice to humans, an acute disease known as hemorrhagic fever with renal syndrome (HFRS, Korean hemorrhagic fever) results. Seoul virus and Puumala virus, which are found later, also members of the genus Hantavirus. Epidemic hemorrhagic fever recognized in Asian countries is caused by Hantaan virus or Seoul virus, while HFRS recognized in European countries is caused by Puumala virus. Puumala virus is carried by rodent hosts indigenous to the European world, for example bank voles (*Clethrionomys glareolus*). Recently, there was recognized hantavirus pulmonary syndrome (HPS) in the U.S, which shows a high mortality. HPS is caused by Sin Nombre virus which is the same phenotype but different antigenically from Hantaan virus.

Main clinical features of epidemic hemorrhagic fever recognized in Asia and caused by Hantaan virus or Seoul virus, are fever, hemorrhage (gastrointestinal, subconjunctival) and renal insufficiency syndrome. By contrast, HFRS recognized in Europe also shows fever, less severe hemorrhage and renal insufficiency syndrome. HPS in U.S. shows syndromes of fever, acute respiratory distress and pulmonary edema, leading to death within about one week after onset of the disease.

There is a great antigenic and genetic diversity among these hantaviruses in nature, needing a respective vaccine for nearly every individual species. Further, high cross-reactivity between each species of genus *hantavirus* makes it difficult to precisely diagnose an infection by certain species of hantanvirus from other hantaviral infections.

Puumala virus infections have been recognized throughout the European countries, and in particular several thousand cases have been reported in North European area annually. While its clinical symptoms are less severe than other hantaviral infections, its respiratory distress and renal insufficiency syndromes make it difficult to discriminate this infection from other respiratory distresses or acute nephritis. Therefore, new precise, rapid and simple serologic diagnosis for Puumala virus has been needed.

### SUMMARY OF THE INVENTION

Accordingly, an object of the present invention is to provide a simple, rapid and effective diagnostic reagent for detecting Puumala virus infection.

The present invention provides a diagnostic reagent for detecting Puumala virus infection which comprises water-insoluble carrier particles and Puumala virus antigen bound to the outer surface thereof.

The present invention further provides a reagent for detecting Puumala virus infection by contacting the reagent to a serum from a subject suspected to have Puumala virus infection and observing agglutination to determine whether the serum contains immunological antibodies against Puumala virus, wherein said reagent comprises sensitized water-insoluble carrier particles which have Puumala virus antigens bound to the outer surface thereof in an amount of 0.005 to 2 wt%.

### DETAILED EXPLANATION OF THE INVENTION

For the present invention, pure Puumala virus antigen obtained from tissue cultured cells or animals is bound to water-insoluble carrier particles to give sensitized carrier particles.

The pure Puumala virus antigen may be obtained from various sources such as organs, in particular brains of animals or tissue-culture cells, which are infected with the virus. In one representative embodiment, Vero E6 cells are inoculated with Puumala virus ATCC VR-984 and cultivated in a suitable manner, and the infected cells are lysed. Virus antigen is isolated and purified from the lysate for use in preparing the diagnostic reagent of the present invention. In other embodiment in which an appropriate animal is used as source of the virus antigen, brain of infant hamster (e.g., before one day-old) is injected with Puumala virus ATCC VR-984, followed by breeding the infected hamsters, disintegrating its brain, and isolating and purifying the virus antigen by ultracentrifugation. The virus antigen is used for sensitizing water-insoluble carrier particles to prepare the diagnostic reagent of the present invention.

The virus antigen is irradiated by γ-ray and chemically modified to improve its reactivity or sensitivity by, for example treating with about 0.05%(v/v) of formalin at low temperature such as 4°C for several days such as 2 weeks to modify the antigen protein. Thus treated antigen is bound to the outer surface of water-insoluble carrier particles such as HDPs (high density particles) or erythrocytes to give sensitized carrier particles. The diagnostic reagent of the present invention comprises an aqueous suspension containing the sensitized carrier particles.

Water-insoluble carrier particles may include known particulate inorganic compounds or animal red blood cells. Particulate inorganic compounds may include, but not limited to, oxides of metals of the family III, IV or VI in element periodic table, for example silica, alumina, titania, zirconia, ferric oxide (Fe₂O₃), iron oxide (Fe₃O₄), cobalt oxide or nickel oxide; hydroxides such as aluminum hydroxide, ferric hydroxide (Fe(OH)₃) or chromium hydroxide; halides such as silver chloride or silver bromide; sulfides such as cadmium sulfide; carbonates such as calcium carbonate or magnesium carbonate; or sulfates such as barium sulfate or strontium sulfate. Among them, silica, alumina, titania, zirconia or complex oxides of these oxides are preferable employed.

Particulate inorganic compounds also may include inorganic oxides comprising silica and oxides of metal selected from the group consisting of the metals of the family II, III and IV in periodic table, in which the oxides of metal can form a complex with silica. Representative examples of the inorganic oxides are lithium oxide, sodium oxide, potassium oxide, magnesium oxide, calcium oxide, strontium oxide, barium oxide, aluminum oxide, titanium oxide, zirconium oxide, germanium oxide, hafnium oxide, tin oxide, zinc oxide and the like, but not limited thereto.

For the present invention, particulate inorganic compounds coated with pigments also may be employed as long as the pigments do not adversely affect the structure of the inorganic compounds. The compounds may be coated with one or more layers of pigments.

The particulate inorganic compounds may have an average particle diameter in the range of 0.1 to 10.0 µm and preferably 0.8 to 5.0 µm, and a specific gravity in the range of 1.5 to 4.0 and preferably from 1.8 to 2.5.

Particulate inorganic complex of multi-coatings may be preferably employed. The particulate inorganic complexes may be prepared by coating the particulate inorganic compounds, for example silica-based inorganic compound particles with a pigment layer, and if necessary with an additional layer of the above-mentioned complex oxides thereon. Then the resulting particles are treated with coupling agents such as silane- or titane-coupling agents to give a reactive surface. Thus obtained particles are usually called 'high density particles (HDPs)' and have an average diameter of 0.1µm to 10.0µm and a specific gravity of 1.5 to 4.0.

For the present invention, the carrier particles have a dispersibility of 80% or more, and preferably 90% or more. The term of 'dispersibility' as used herein indicates the percentage of number of individual non-agglomerated particles among the number of total particles when suspended in an aqueous medium. The dispersibility may be measured with a commonly used counter, for example Coltercounter Model ZD-1.

The carrier particles may have various shapes depending on the crystal structure of the inorganic compounds used and their production method. They usually are in the shape of polyhedral, columns, cones or spheres. Spheres, in particular real spheres are preferably used among others. These particles may be prepared by known methods described in, for example JP 52-138094A or JP 61-149644A.

It is possible to use the carrier particles impregnated with known dyes. The dyes may include, but not limited to, cationic dyes such as malachite green, rhodamine B or methylene blue; dispersion dyes such as dianix™ (Mitsubishi Chemical Co., Ltd., Japan), disbasol™ (I.C.I., U.K.) or miketone Polyester™ (Mitsui Toatsu K.K. Japan); direct dyes such as Congo red, direct deep black E W or crysophenin G; alizarin saphrol B; acid dyes such as alizarin direct blue A or alizarin ciarine green G; acid dyes such as diamond black F, cromfast navyblue B or palatine fast blue BN; metal-containing dyes such as asidol (BASF, Germany), eisen opal (Horoya Kagakusa, Japan) or oreosol (Taoka Kagakusa, Japan); reactive dyes such as diamira, michishiron (Mitsubishi Chemical Co., Ltd., Japan) or sumifix (Sumitomo Chemical Co., Ltd., Japan); fluorescent brightening dyes such as mikiwhite (Mitsubishi Chemical Co., Ltd., Japan) or whitux (Sumitomo Chemical Co., Ltd., Japan). Preferred are Metal-containing dyes and cationic dyes, and more preferred are cationic dyes.

Those dyes having a solubility of 1 parts by weight or more, more particularly 5 parts by weight, and most preferably 10 parts by weight in 100 parts of methanol are employed.

The amount of dyes in the carrier particles may be in the range of 0.5 wt% to 8 wt% and preferably 1.0 wt% to 5.0 wt%, but not limited thereto. Within the above-mentioned range, the dyes enable an easy observation of antigen-antibody agglutination for detecting Puumala virus infection as well as prevent effluent of the dye from the inorganic compound carrier particles.

Blood cells also may be used as water-insoluble carrier particles for Puumala virus antibody detection according to the present invention. The source or type of the blood cells is not particularly limited and may be selected from those employed as carriers for antigen-antibody agglutination detection. Although the blood cells show a relatively low sensitivity than HDPs, they still may be advantageously used as water-insoluble carrier particles for the present invention.

The carrier particles sensitized with Puumala virus antigen may be prepared contacting the virus antigen produced as described above with the carrier particles so as to bind the antigen to the outer surface of the particles. The method of binding the antigen to the particles is not particularly limited, but commonly employed methods can be used. For example, the particles are contacted with the antigen in an aqueous medium such as physiological saline or phosphate buffered saline (PBS) by mixing the antigen with a suspension of the particles in an aqueous medium.

The ratio of the antigen to the carrier particle is in the range of 100 to 400 HDP agglutination units, and preferably 150 to 250 HDP agglutination unit per 1g of particle. The contact is performed at pH 6.0 - 8.0 and at a temperature of 4°C - 20°C. The term of 'HDP agglutination unit' used herein is defined as the smallest amount of the antigen required for HDP-sera agglutination detection.

After the mixing, the unbound antigens are removed by washing and the solution was further incubated with an inert serum protein such as bovine serum albumin (BSA) to saturate the remaining active sites of the outer surface of the particles.

The diagnostic reagent according to the present invention may be prepared by suspending the sensitized particles in an aqueous solution to a concentration of 0.005 to 2 wt%, and preferably 0.05 to 1 wt%.

The sensitized carrier particles may be lyophilized to prolong its shelf life although the particles themselves are stable, and may be used by just suspending the lyophilized carrier particles into an appropriate aqueous medium, e.g., distilled water just before use.

The diagnostic reagent of the present invention is reactive to sera sample from a patient suspected to have or prone to infection with Puumala virus to result in agglutinations with high specificity and sensitivity.

### Examples

The present invention will be described in more detail by way of various Examples, which should not be construed to limit the scope of the present invention. In particular, water-insoluble carrier particles are prepared by coating silica core with a mixture of dye and silica and then subjecting to surface-treatment with a silane coupling agent as one embodiment of the present invention, which should not be construed to limit the kind of the water-insoluble carrier particles.

### Example 1 : Preparation of Puumala virus antigen

Sotkamo strain of Puumala virus ATCC VR-984 was obtained from American Type Culture Collection (MD, USA) and used as a source of Puumala virus antigen.

Puumala virus ATCC VR-984 was passaged in Vero E6 cells (ATCC CRL 1586) until 10th passage and the clones forming the largest and most clear plaques are harvested for use as seed virus. The preparation of virus antigen is performed by using Vero E6 cells or an infant hamster until one day after birth as host. Vero E6 cell is preferably used.

Preparation of virus antigen using Vero E6 cell or infant hamster are explained below.

### (1) Growth of virus in Vero E6 cells

Tissue-cultured Vero E6 cells were inoculated with 0.1 ml of Puumala virus obtained in the above and cultivated in a CO₂ incubator at 37°C for about 10 days. The infected cells were treated with 0.1% Triton-X and harvested. The harvested cells were disintegrated with a sonicator and centrifuged at 4°C to give a supernatant containing virus particles. The supernatant was treated with gamma-ray of 3000 rad to completely inactivate the virus. The inactivated virus was inoculated into Vero E6 cells and subjected to indirect fluorescent antibody (IFA) assay to confirm the virus inactivation. Ultracentrifugation was carried out at 70,000 rpm at 4°C for 4 hours to give a supernatant containing virus antigen, which was diluted with PBS and filtered through an ultrafilter to obtain purified Puumala virus antigen.

Thus obtained Puumala virus antigen solution was treated with 0.05%(v/v) of formalin at 4°C for 2 weeks to modify the surface antigen protein to improve the sensitivity. The antigen was dialyzed over physiological saline and used as antigen component for the diagnostic reagent of the present invention.

### (2) Growth of virus infant hamster

Puumala virus (0.01 nil) was injected into brain of infant hamster (no later than one day old). About 20 days later, when more than half of the animals show paralysis, animal brain was removed aseptically and homogenized. Physiological saline was added to the homogenate to yield an emulsion (10-20%(v/v)). Thus obtained brain emulsion was centrifuged at 600 rpm, 4°C for 30 minutes, and ethyl alcohol of 10 tunes by volume was added to the supernatant, followed by gentle shaking at low temperature and drying under reduced pressure.

Physiological saline was added to the dried brain to its original volume and 20%(v/v) protamine sulfate was added. After gentle shaking at low temperature, centrifugation was performed at 10,000 rpm for 60 minutes to give a supernatant containing virus antigen free of basic myelin protein. The supernatant was diluted with PBS and filtered through an ultrafilter to obtain purified Puumala virus antigen.

Thus obtained Puumala virus antigen solution was treated with 0.05%(v/v) of formalin at 4°C for 2 weeks to modify the surface antigen protein to improve the sensitivity. The antigen was dialyzed over physiological saline and used as antigen component for the diagnostic reagent of the present invention.

The titer of thus-obtained antigen solution was measured by ELISA so that the diagnostic reagent containing the antigen can have a constant titer. The titer of the antigen solution obtained in (1) and (2) are 5 - 10 HDP agglutination units/nil and 120 HDP agglutination units/ml, respectively.

### Example 2 : Preparation of high density particles

To a glass flask equipped with stirrer were added 2800 ml of methanol, 616 ml of aqueous ammonia (25 wt%) and 21 ml of aqueous sodium hydroxide solution (5M). 256 ml of solution (22 wt%) of tetraethyl silicate in methanol was added dropwise at 10°C with a rate of 25.5 ml/hr to give silica particles having a diameter of 0.91µm. 625 ml of solution (44 wt%) of tetraethyl silicate in methanol and 625 ml of solution (1.25 wt%) of methylene blue in methanol were added dropwise simultaneously at a rate of 25.5 ml/hr to give colored silica particles. The silica particles were repeatedly suspended and decanted to carry out purification as well as washing.

Thus obtained two-layered silica/dye complex particles have an average diameter of about 1.57 µm. The carrier particles were suspended in methanol to a concentration of 0.5 wt%, and phenyltriethoxy silan was added to the resulting suspension (100 ml) to a concentration of 0.5 wt%. After reaction of 10°C for 16 hours, surface-modified silica particles were obtained.

### Example 3 : Preparation of Puumala virus antigen coated-HDP

M/60 and PBS (pH 7.2) were added to Puumala virus antigen solution obtained in Example 1 to dilute the solution to 2 HDP agglutination units/nil. The resulting antigen solution (5 ml) was mixed with 0.5% HDP/PBS (5 ml) and sensitization was carried out with gentle shaking for 60 minutes. Then, the mixture was washed with PBS three tunes, and suspended in a dilution (5 ml) of immobilized rabbit serum in PBS at 56°C for 30 minutes to a concentration of 1% to give sensitized HDP solution.

### Example 4 : Measurement of antibody titer using HDPA or IFA

The antibody titer of the sera from HFRS patients are measured by using HDPA (High Density Particle Agglutination) as well as indirect IFA (Immuno-Fluorescent Assay).

Each 0.025 ml of the dilution containing in Example 3 is distributed into the wells of V-shaped microplate, and 0.025 ml of serum (1:10 dilution) to be tested was added to the first well. The test sample was serially diluted and the sensitized HDP solution (0.025 ml) in Example 3 is added dropwise and mixed well. After allowing to stand at room temperature for 40 minutes, agglutination was observed for every dilution and the maximum dilution which showed antigen-antibody agglutination was defined as 'HDP antibody titer.' By following the same procedure, HDP antibody titer were measured for 43 test serum samples.

Besides, conventional IFA (indirect Immuno-fluorescent antibody assay) was performed to 43 test sera samples, and the maximum dilution which showed fluorescent antibody-antigen complex was defined as 'IFA antibody titer.'

For comparison, the sensitivity and specificity of IFA and HDPA against the Hantavirus infection were determined by using the diagnostic reagent disclosed in KR 90-16761A, which comprises HDPs sensitized with Hantaan virus.

The results are shown in Table 1.

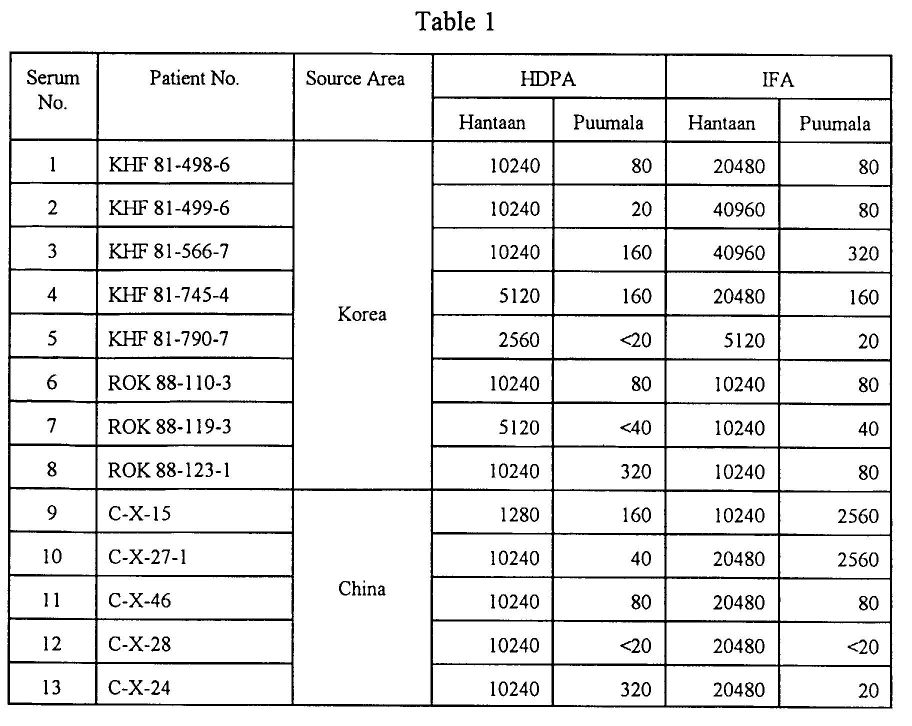

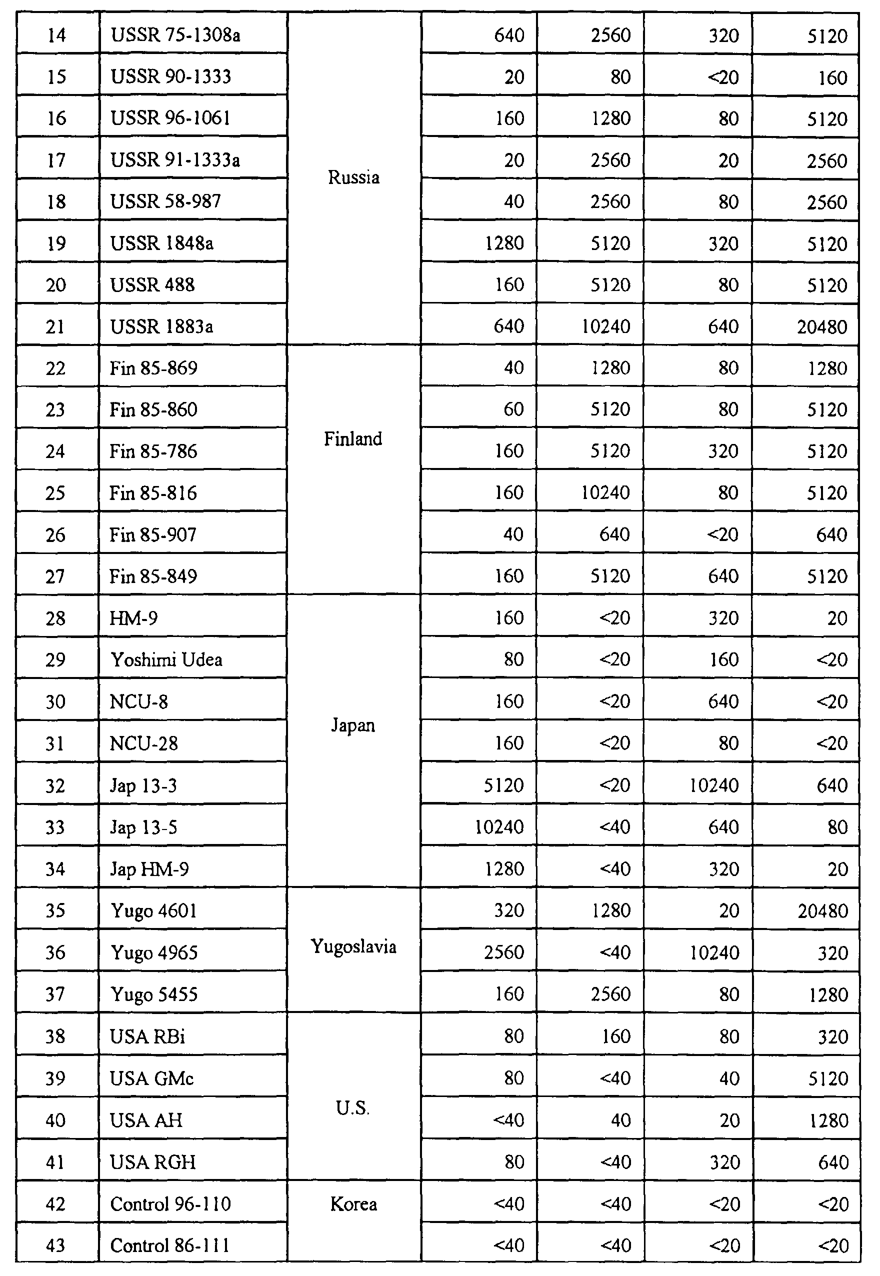

As shown in Table 1, the results of IFA and HDPA showed that the patients in Korea, Japan and China had Hantaan virus infection while those in Russia, Finland and Yugoslavia had Puumala virus infection. IFA and HDPA showed no significant difference in sensitivity and specificity. The diagnostic reagent of the present invention allows a simple test to detect and discriminate Puumala virus infection from Hantaan virus infection with sensitivity as high as of conventional IFA. Therefore, it is possible to diagnose in an early stage of hemorrhagic fever with renal syndrome (HFRS) caused by Puumala virus infection from its related syndromes such as acute respiratory distress or renal syndromes, which are hard to discriminate from the HFRS by a clinical examination.

### Example 5 : Preparation of erythrocytes sensitized with Puumala virus antigen

To PBS containing 2.5% immobilized sheep erythrocytes was added an equivalent volume of 1:100,000 tannic acid/PBS solution, and the mixture was allowed to react at 20°C for 20 minutes. The reaction mixture was washed with PBS (a mixture of 1/60 mole phosphate buffered saline (pH 7.2) and physiological saline in 1:3 volume) once and then suspended into PBS to prepare 0.5% suspension containing tannin-blood cell complex.

The suspension of tannin-blood cell was mixed with 80 times dilution of Puumala virus antigen obtained in Example 1 (1 : 1 volume) and the resulting mixture was gently shaken at room temperature for 60 minutes to perform sensitization. Then, the mixture was washed with PBS three times, and suspended in a dilution of immobilized rabbit serum (1%) in PBS to a concentration of 0.5% to give sensitized erythrocyte solution.

### Example 6 : Measurement of antibody titer using sensitized erythrocyte

Each 0.025 ml of the dilution containing immobilized rabbit serum (Example 3) is distributed into the wells of V-shaped microplate, and 0.025 ml of serum (1:10 dilution; Serum No. 11 in Table 1) was added to the first well. The test sample was serially diluted and the sensitized erythrocyte solution (0.025 ml) in Example 3 is added dropwise and mixed well. After allowing to stand at room temperature for 3 hours, agglutination was observed. As a result thereof, the antibody titer was 40.

Although preferred embodiments of the present invention have been described in detail herein above, it should be clearly understood that many variations and/or modifications of the basic inventive concepts herein taught which may appear to those skilled in the art will still fall within the spirit and scope of the present invention as defined in the appended claims.

## Claims

1. A reagent for detecting Puumala virus infection by bringing virus sensitized particles contained in the reagent to a contact with a serum from a subject suspected to have Puumala virus infection and observing agglutination to determine whether the serum contains antibodies against Puumala virus, wherein said particles are sensitized water-insoluble carrier particles which have Puumala virus antigens bound to the outer surface thereof in an amount of 0.005 to 2 wt%.

2. A reagent for detecting Puumala virus infection as claimed in Claim 1, wherein said sensitized water-insoluble carrier particles are suspended in phosphate buffered saline (PBS) containing 1%(v/v) immobilized rabbit sera.

3. A reagent for detecting Puumala virus infection as claimed in Claim 1 or Claim 2, wherein said Puumala virus antigens are obtained from tissue-cultured Vero E6 cells or brain of hamster

4. A reagent for detecting Puumala virus infection as claimed in Claim 1 or 2, wherein said water-insoluble carrier particles are inorganic compound particles or erythrocytes.

5. A reagent for detecting Puumala virus infection as claimed in Claim 4, wherein said inorganic compound particles are selected from the group consisting of oxides of metals of the family III, IV or VI in element periodic table, selected from the group consisting of silica, alumina, titania, zirconia, ferric oxide (Fe₂O₃), iron oxide (Fe₃O₄), cobalt oxide and nickel oxide; hydroxides selected from the group consisting of aluminum hydroxide, ferric hydroxide (Fe(OH)₃) and chromium hydroxide; halides selected from the group consisting of silver chloride and silver bromide; sulfides; carbonates selected from the group consisting of calcium carbonate and magnesium carbonate; or sulfates selected from the group consisting of barium sulfate and strontium sulfate

6. A reagent for detecting Puumala virus infection as claimed in Claim 4, wherein said inorganic compound particles are selected from the group consisting of silica and oxides of metal selected from the group consisting of the metals of II, III and IV family in periodic table, said oxides being capable of attaching to the silica.

7. A reagent for detecting Puumala virus infection as claimed in Claim 4, wherein said inorganic compound particles are high density particles prepared by coating inorganic compound particles with dyes or mixtures of dyes and inorganic compounds, and treated with silane- or titan-coupling agents to have a reactive surface, and said heavy density particles have an average particle diameter in the range of 0.1 to 10.0 µm and a specific gravity in the range of 1.5 to 4.0.

8. A reagent for detecting Puumala virus infection as claimed in Claim 7, wherein said dyes are selected from the group consisting of cationic dyes, dispersion dyes, direct dyes, acid dyes, acid dyes, metal-containing dyes and fluorescent brightening dyes.

9. A reagent for detecting Puumala virus infection as claimed in Claim 1, wherein said antigens are pretreated, before bounding to the particles, with formalin at low temperature to improve their sensitivity.
